# EUROPÄISCHE PATENTSCHRIFT

(11) **EP 0 937 075 B1**
(45) Veröffentlichungstag und Bekanntmachung des Hinweises auf die Patenterteilung: **17.12.2003**
(21) Anmeldenummer: 97950116.0
(22) Anmeldetag: 05.11.1997
(51) Int. Cl.: C07D 471/04, C07D 491/04, A61K 31/435

(54) **Neue Imidazo- und Oxazolopyridine als Phosphodiesterase Inhibitoren.**
New Imidazo- and Oxazolopyridines as Phosphodiesterase Inhibitors.
Nouvelles Imidazo- et Oxazolopyridines comme inhibiteurs de la Phosphodiesterase.

(30) Priorität: 11.11.1996 DE 19646460; 13.11.1996 EP 96118187
(43) Veröffentlichungstag der Anmeldung: 25.08.1999
(73) Patentinhaber: ALTANA Pharma AG, 78467 Konstanz (DE)
(72) Erfinder: AMSCHLER, Hermann, (DE); MARTIN, Thomas, D-78462 Konstanz (DE); FLOCKERZI, Dieter, D-78476 Allensbach (DE); SCHMIDT, Beate, D-78476 Allensbach (DE); THIBAUT, Ulrich, D-78464 Konstanz (DE); HATZELMANN, Armin, D-78467 Konstanz (DE); BOSS, Hildegard, D-78476 Allensbach (DE); HÄFNER, Dietrich, D-78464 Konstanz (DE); KLEY, Hans-Peter, D-78476 Allensbach (DE); BEUME, Rolf, D-78465 Konstanz (DE); BÄR, Thomas, D-78479 Reichenau (DE); ULRICH, Wolf-Rüdiger, D-78464 Konstanz (DE)
(86) Internationale Anmeldenummer: EP9706130
(87) Internationale Veröffentlichungsnummer: WO98021209

(56) Entgegenhaltungen:
- EP-A- 0 079 083
- WO-A-94/12461
- WO-A-95/14680
- WO-A-95/35282
- WO-A-96/11917
- US-A- 4 038 396

## Beschreibung

### Anwendungsgebiet der Erfindung

Die Erfindung betrifft neue Oxazolo- sowie Imidazopyridine, die in der pharmazeutischen Industrie zur Herstellung von Medikamenten verwendet werden.

### Bekannter technischer Hintergrund

In den deutschen Patentanmeldungen DE 23 30 109, DE 25 27 321, DE 26 19 547 und DE 26 33 905 sowie dem amerikanischen Patent US 4,038,396 werden 2-(substituiertes Phenyl)-oxazolo[4,5-b]pyridine, -oxazolo[4,5-c]pyridine und -oxazolo[5,4-b]pyridine als antiinflammatorische, antipyretische und analgetische Verbindungen beschrieben.

In der deutschen Patentanmeldung DE 23 61 757 werden 2-(substituiertes Phenyl)-imidazo-[4,5-b]pyridine mit blutdrucksenkenden, positiv inotropen, die Thrombozytenaggregation hemmenden und die Blutungszeit verlängernden Eigenschaften beschrieben.

In der europäischen Patentanmeldung EP 022 495 werden 2-(substituiertes Phenyl)-imidazo[4,5-b]-pyridine als Kardiotonika und hypertensive Agenzien beschrieben. in der europäischen Patentanmeldung EP 072 926 werden 2-(substituiertes Phenyl)-imidazo[4,5-b]- und -[4,5-c]pyridine mit positiv inotropen Eigenschaften beschrieben. In der europäischen Patentanmeldung EP 079 083 werden 2-Phenyl-imidazo[4,5-c]pyridine mit vasodilatierenden, positiv inotropen und die Thrombozytenaggregation hemmenden Eigenschaften beschrieben, die keine hemmende Wirkung auf die myokardiale Phosphodiesterase besitzen.

In der PCT-Anmeldung WO94/12461 werden unter anderem 2-(substituiertes Phenyl)-imidazopyridine und -oxazolopyridine als selektive Inhibitoren der Phosphodiesterase des Typs 4 beschrieben.

### Beschreibung der Erfindung

Es wurde nun gefunden, daß die nachfolgend näher beschriebenen neuen Verbindungen der allgemeinen Formel I überraschende und besonders vorteilhafte Eigenschaften besitzen.

Gegenstand der Erfindung sind somit Verbindungen der Formel I, 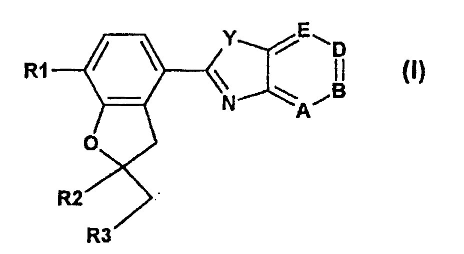 worin
aus der Gruppe der Symbole A, B, D und E unabhängig voneinander ein Symbol für N (Stickstoff) steht, ein Symbol für C-X und die verbleibenden zwei Symbole für C-H stehen, wobei
- X: Wasserstoff, Hydroxy, Halogen, Cyano, Nitro, Carboxyl, 1-4C-Alkyl, 1-4C-Alkylcarbonyl, 1-4C-Alkylcarbonyloxy, 1-4C-Alkoxy, 1-4C-Alkoxycarbonyl, Amino, Mono- oder Di-1-4C-alkylamino, 1-4C-Alkylcarbonylamino, Hydroxy-1-4C-alkyl, Hydroxysulfonyl, 1-4C-Alkylsulfonyl, 1-4C-Alkoxysulfonyl oder Sulfamoyl bedeutet,
- Y: O (Sauerstoff) oder NH bedeutet,
- R1: 1-4C-Alkoxy, 3-7C-Cycloalkoxy, 3-7C-Cycloalkylmethoxy, Benzyloxy oder ganz oder überwiegend durch Fluor substituiertes 1-4C-Alkoxy bedeutet,
- R2: Wasserstoff oder 1-4C-Alkyl und
- R3: Wasserstoff oder 1-4C-Alkyl bedeuten,
oder worin
- R2 und R3: gemeinsam und unter Einschluß der beiden Kohlenstoffatome, an die sie gebunden sind, einen spiro-verknüpften 5-, 6- oder 7-gliedrigen, gewünschtenfalls durch ein Sauerstoffatom unterbrochenen Kohlenwasserstoffring darstellen,
sowie die Salze und die N-Oxide dieser Verbindungen.

Halogen im Sinne der Erfindung sind Fluor, Chlor, Brom und Jod.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-4C-Alkylcarbonyl steht für eine Carbonylgruppe, an die einer der vorstehend genannten 1-4C-Alkylreste gebunden ist. Beispielsweise sei der Acetylrest (CH₃CO-) genannt.

1-4C-Alkylcarbonyloxyreste enthalten neben dem Sauerstoffatom einen der vorstehend genannten 1-4C-Alkylcarbonylreste. Beispielsweise sei der Acetoxyrest (CH₃CO-O-) genannt.

1-4C-Alkoxy steht für einen Rest, der neben dem Sauerstoffatom einen geradkettigen oder verzweigten Alkylrest mit 1 bis 4 Kohlenstoffatomen enthält. Als Alkoxyreste mit 1 bis 4 Kohlenstoffatomen seien beispielsweise genannt der Butoxy-, iso-Butoxy-, sec.-Butoxy-, tert.-Butoxy-, Propoxy-, isopropoxy-, Ethoxy- und der Methoxyrest.

1-4C-Alkoxycarbonyl steht für eine Carbonylgruppe, an die einer der vorstehend genannten 1-4C-Alkoxyreste gebunden ist. Beispielsweise seien der Methoxycarbonyl- (CH₃O-CO-) und der Ethoxycarbonylrest (CH₃CH₂O-CO-) genannt.

Als Mono- oder Di-1-4C-alkylaminoreste seien beispielsweise der Methylamino-, der Dimethylamino- und der Diethylaminorest genannt.

Als 1-4C-Alkylcarbonylaminorest sei beispielsweise der Acetylaminorest (-NH-CO-CH₃) genannt.

Hydroxy-1-4C-alkyl steht für vorstehend genannte 1-4C-Alkylresle, die durch eine Hydroxylgruppe substituiert sind. Beispielsweise seien der Hydroxyethyl- und der Hydroxymethylrest genannt.

1-4C-Alkylsulfonyl steht für eine Sulfonylgruppe, an die einer der vorstehend genannten 1-4C-Alkylreste gebunden ist. Beispielsweise sei der Methylsulfonylrest (CH₃SO₂-) genannt.

1-4C-Alkoxysulfonyl steht für eine Sulfonylgruppe, an die einer der vorstehend genannten 1-4C-Alk- oxyreste gebunden ist. Beispielsweise seien der Methoxysulfonyl- (CH₃O-SO₂-) und der Ethoxysulfonylrest (CH₃CH₂O-SO₂-) genannt.

3-7C-Cycloalkoxy steht für den Cyclopropyloxy-, Cyclobutyloxy-, Cyclopentyloxy-, Cyclohexyloxy- und Cycloheptyloxyrest. Bevorzugt seien die 3-5C-Cycloalkyloxyreste Cyclopropyloxy, Cyclobutyloxy und Cyclopentyloxy genannt.

3-7C-Cycloalkylmethoxy steht für Cyclopropylmethoxy, Cyclobutylmethoxy, Cyclopentylmethoxy, Cyclohexylmethoxy und Cycloheptylmethoxy. Bevorzugt seien die 3-5C-Cycloalkylmethoxyreste Cyclopropylmethoxy, Cyclobutylmethoxy und Cyclopentylmethoxy genannt.

Als ganz oder überwiegend durch Fluor substituiertes 1-4C-Alkoxy seien beispielsweise der 2,2,3,3,3-Pentafluorpropoxy-, der Perfluorethoxy-, der 1,2,2-Trifluorethoxy- und insbesondere der 1,1,2,2-Tetrafluorethoxy-, der 2,2,2-Trifluorethoxy-, der Trifluormethoxy- und bevorzugt der Difluormethoxyrest genannt.

Als spiro-verknüpfter 5-, 6- oder 7-gliedriger, gewünschtenfalls durch ein Sauerstoffatom unterbrochener Kohlenwasserstoffring sei der Cyclopentan-, der Cyclohexan-, der Cycloheptan-, der Tetrahydrofuran- und der Tetrahydropyranring genannt.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Dem Fachmann ist bekannt, daß die erfindungsgemäßen Verbindungen als auch ihre Salze, wenn sie zum Beispiel in kristalliner Form isoliert werden, verschiedene Mengen an Lösungsmitteln enthalten können. Die Erfindung umfaßt daher auch alle Solvate und insbesondere alle Hydrate der Verbindungen der Formel I, sowie alle Solvate und insbesondere alle Hydrate der Salze der Verbindungen der Formel I.

Hervorzuhebende Verbindungen der Formel I sind solche, worin
aus der Gruppe der Symbole A, B, D und E unabhängig voneinander ein Symbol für N (Stickstoff) steht, ein Symbol für C-X und die verbleibenden zwei Symbole für C-H stehen, wobei
- X: Wasserstoff, Hydroxy, Halogen, Cyano, Nitro, Carboxyl, 1-4C-Alkyl, 1-4C-Alkylcarbonyloxy, 1-4C-Alkoxy, 1-4C-Alkoxycarbonyl, Amino, Mono- oder Di-1-4C-alkylamino, 1-4C-Alkylcarbonylamino, Hydroxy-1-4C-alkyl, Hydroxysulfonyl oder Sulfamoyl bedeutet,
- Y: O oder NH bedeutet,
- R1: 1-4C-Alkoxy, 3-7C-Cycloalkoxy, 3-7C-Cycloalkylmethoxy oder ganz oder überwiegend durch Fluor substituiertes 1-4C-Alkoxy bedeutet,
- R2: 1-4C-Alkyl und
- R3: Wasserstoff oder 1-4C-Alkyl bedeuten,
oder worin
- R2 und R3: gemeinsam und unter Einschluß der beiden Kohlenstoffatome, an die sie gebunden sind, einen spiro-verknüpften Cyclopentan-, Cyclohexan-, Tetrahydrofuran- oder Tetrahydropyranring darstellen,
sowie die Salze dieser Verbindungen.

Besonders hervorzuhebende Verbindungen der Formel I sind solche, worin
aus der Gruppe der Symbole A, B, D und E unabhängig voneinander ein Symbol für N (Stickstoff) steht, ein Symbol für C-X und die verbleibenden zwei Symbole für C-H stehen, wobei
- X: Wasserstoff, Halogen, Carboxyl oder 1-4C-Alkoxycarbonyl bedeutet,
- Y: O oder NH bedeutet,
- R1: Methoxy, Ethoxy, Cyclopropylmethoxy oder ganz oder überwiegend durch Fluor substituiertes 1-2C-Alkoxy bedeutet,
- R2: 1-4C-Alkyl und
- R3: Wasserstoff oder 1-4C-Alkyl bedeuten,
oder worin
- R2 und R3: gemeinsam und unter Einschiuß der beiden Kohlenstoffatome, an die sie gebunden sind, einen spiro-verknüpften Cyclopentan- oder Cyclohexanring darstellen,
sowie die Salze dieser Verbindungen.

Bevorzugte Verbindungen der Formel I sind solche, in denen
aus der Gruppe der Symbole A, B, D und E unabhängig voneinander ein Symbol für N (Stickstoff) steht, ein Symbol für C-X und die verbleibenden zwei Symbole für C-H stehen, wobei
- X: Wasserstoff oder Halogen bedeutet,
- Y: O oder NH bedeutet,
- R1: Methoxy bedeutet und
- R2 und R3: gemeinsam und unter Einschluß der beiden Kohlenstoffatome, an die sie gebunden sind, einen spiro-verknüpften Cyclopentan- oder Cyclohexanring darstellen,
sowie die Salze dieser Verbindungen.

Beispielhafte erfindungsmäßige Verbindungen sind in den folgenden Tabellen aufgeführt:

**Tabelle 1**

| Verbindungen der Formel I mit Y=O, A=N, B=CH, D=CH, E=CH und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C₃H₅ | CH₂CH₂-O | |
| OCF₂H | CH₂CH₂-O | |
| OCF₃ | CH₂CH₂-O | |
| OCH₂CF₃ | CH₂CH₂-O | |
| OCH₃ | CH₂CH₂-O-CH₂ | |
| OC₂H₅ | CH₂CH₂-O-CH₂ | |
| OCH₂C₃H₅ | CH₂CH₂-O-CH₂ | |
| OCF₂H | CH₂CH₂-O-CH₂ | |
| OCF₃ | CH₂CH₂-O-CH₂ | |
| OCH₂CF₃ | CH₂CH₂-O-CH₂ | |

**Tabelle 2**

| Verbindungen der Formel I mit Y=O, A=CH, B=N, D=CH, E=CH und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C₃H₅ | CH₂CH₂-O | |
| OCF₂H | CH₂CH₂-O | |
| OCF₃ | CH₂CH₂-O | |
| OCH₂CF₃ | CH₂CH₂-O | |
| OCH₃ | CH₂CH₂-O-CH₂ | |
| OC₂H₅ | CH₂CH₂-O-CH₂ | |
| OCH₂C₃H₅ | CH₂CH₂-O-CH₂ | |
| OCF₂H | CH₂CH₂-O-CH₂ | |
| OCF₃ | CH₂CH₂-O-CH₂ | |
| OCH₂CF₃ | CH₂CH₂-O-CH₂ | |

**Tabelle 3**

| Verbindungen der Formel I mit Y=O, A=CH, B=CH, D=N, E=CH und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ H | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C₃H₅ | CH₂CH₂-O | |
| OCF₂H | CH₂CH₂-O | |
| OCF₃ | CH₂CH₂-O | |
| OCH₂CF₃ | CH₂CH₂-O | |
| OCH₃ | CH₂CH₂-O-CH₂ | |
| OC₂H₅ | CH₂CH₂-O-CH₂ | |
| OCH₂C₃H₅ | CH₂CH₂-O-CH₂ | |
| OCF₂H | CH₂CH₂-O-CH₂ | |
| OCF₃ | CH₂CH₂-O-CH₂ | |
| OCH₂CF₃ | CH₂CH₂·O-CH₂ | |

**Tabelle 4**

| Verbindungen der Formel I mit Y=O, A=CH, B=CH, D=CH, E=N und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C-₃H₅ | CH₂CH₂-O | |
| OCF₂H | CH₂CH₂-O | |
| OCF₃ | CH₂CH₂-O | |
| OCH₂CF₃ | CH₂CH₂-O | |
| OCH₃ | CH₂CH₂-O-CH₂ | |
| OC₂H₅ | CH₂CH₂-O-CH₂ | |
| OCH₂C₃H₅ | CH₂CH₂-O-CH₂ | |
| OCF₂H | CH₂CH₂-O-CH₂ | |
| OCF₃ | CH₂CH₂-O-CH₂ | |
| OCH₂CF₃ | CH₂CH₂-O-CH₂ | |

**Tabelle 5**

| Verbindungen der Formel I mit Y=NH, A=N, B=CH, D=CH, E=CH und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C₃H₅ | CH₂CH₂-O | |
| OCF₂H | CH₂CH₂-O | |
| OCF₃ | CH₂CH₂-O | |
| OCH₂CF₃ | CH₂CH₂-O | |
| OCH₃ | CH₂CH₂-O-CH₂ | |
| OC₂H₅ | CH₂CH₂-O-CH₂ | |
| OCH₂C₃H₅ | CH₂CH₂-O-CH₂ | |
| OCF₂H | CH₂CH₂-O-CH₂ | |
| OCF₃ | CH₂CH₂-O-CH₂ | |
| OCH₂CF₃ | CH₂CH₂-O-CH₂ | |

**Tabelle 6**

| Verbindungen der Formel I mit Y=NH, A=CH, B=N, D=CH, E=CH und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C₃H₅ | CH₂CH₂-O | |
| OCF₂H | CH₂CH₂-O | |
| OCF₃ | CH₂CH₂-O | |
| OCH₂CF₃ | CH₂CH₂-O | |
| OCH₃ | CH₂CH₂-O-CH₂ | |
| OC₂H₅ | CH₂CH₂-O-CH₂ | |
| OCH₂C₃H₅ | CH₂CH₂-O-CH₂ | |
| OCF₂H | CH₂CH₂-O-CH₂ | |
| OCF₃ | CH₂CH₂-O-CH₂ | |
| OCH₂CF₃ | CH₂CH₂-O-CH₂ | |

**Tabelle 7**

| Verbindungen der Formel I mit Y=NH, A=CCI, B=N, D=CH, E=CH und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C₃H₅ | CH₂CH₂₋O | |
| OCF₂H | CH₂CH₂-O | |
| OCF₃ | CH₂CH₂-O | |
| OCH₂CF₃ | CH₂CH₂-O | |
| OCH₃ | CH₂CH₂-O-CH₂ | |
| OC₂H₅ | CH₂CH₂-O-CH₂ | |
| OCH₂C₃H₅ | CH₂CH₂-O-CH₂ | |
| OCF₂H | CH₂CH₂-O-CH₂ | |
| OCF₃ | CH₂CH₂-O-CH₂ | |
| OCH₂CF₃ | CH₂CH₂-O-CH₂ | |

sowie die Salze der in den Tabellen genannten Verbindungen.

Bei den Verbindungen der Formel I kann es sich, wenn Y NH bedeutet, um Tautomeren und - sofern die Substituenten -R2 und -CH₂R3 nicht identisch sind - um chirale Verbindungen handeln. Die Erfindung umfaßt daher sowohl die reinen Tautomeren und Enantiomeren als auch deren Gemische in jedem Mischungsverhältnis, einschließlich der Racemate. Die Enantiomeren können in an sich bekannter Weise (zum Beispiel durch Herstellung und Trennung entsprechender diastereoisomerer Verbindungen) separiert werden.

Bevorzugt sind jedoch die Verbindungen der Formel I, in denen die Substituenten -R2 und -CH₂R3 identisch sind oder in denen R2 und R3 gemeinsam und unter Einschluß der beiden Kohlenstoffatome, an die sie gebunden sind, einen spiro-verknüpften 5-, 6- oder 7-gliedrigen, gewünschtenfalls durch ein Sauerstoffatom unterbrochenen Kohlenwasserstoffring darstellen.

Weiterer Gegenstand der Erfindung sind Verfahren zur Herstellung der Verbindungen der Formel I und ihrer Salze. Das erste Verfahren (vgl. Schema 1) ist dadurch gekennzeichnet, daß man Verbindungen der Formel II, in denen R1, R2 und R3 die oben angegebenen Bedeutungen haben und Z eine geeignete Abgangsgruppe darstellt, mit Verbindungen der Formel III, worin Y O (Sauerstoff) oder NH bedeutet, und A, B, D und E die oben angegebenen Bedeutungen haben, umsetzt.

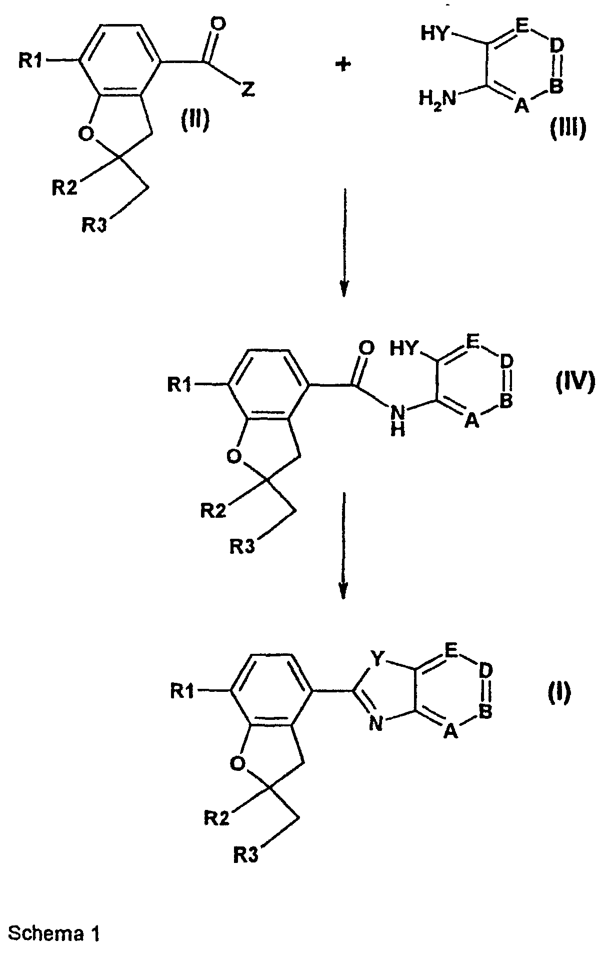

Welche Abgangsgruppen Z geeignet sind, ist dem Fachmann aufgrund seines Fachwissens geläufig. Beispielsweise wird von geeigneten Säurehalogeniden der Formel II (Z = Cl oder Br) ausgegangen.

Die Umsetzung erfolgt bevorzugt in Gegenwart einer Base wie z.B. Pyridin oder Triethylamin, in einem geeigneten inerten Lösungsmittel, z.B. in einem cyclischen Kohlenwasserstoff wie Toluol oder Xylol, oder einem sonstigen inerten Lösungsmittel wie Dioxan oder ohne weiteres Lösungsmittel, vorzugsweise bei erhöhter Temperatur.

Die bei der Umsetzung zunächst entstehenden Verbindungen der Formel IV, worin R1, R2, R3, A, B, D, E und Y die oben angegebenen Bedeutungen besitzen, werden durch innere Kondensation zu entsprechenden Verbindungen der Formel I umgesetzt. Diese innere Kondensation erfolgt bevorzugt in Gegenwart eines geeigneten Kondensationsmittels, wie beispielsweise Thionylchlorid oder Phosphoroxytrichlorid, in einem geeigneten inerten Lösungsmittel oder ohne weiteres Lösungsmittel unter Verwendung eines Überschusses an Kondensationsmittel, vorzugsweise bei erhöhter Temperatur, insbesondere bei der Siedetemperatur des verwendeten Lösungs- bzw. Kondensationsmittels.

Die Umsetzung erfolgt beispielsweise so wie in den nachfolgenden Beispielen beschrieben, oder auf eine dem Fachmann an sich vertraute Weise (z.B. so wie in der deutschen Patentanmeldung DE 23 30 109, im amerikanischen Patent US 4,038,396, in J. Med. Chem. **1978,** 21, (11), 1159 ff., in der europäischen Patentanmeldung EP 072 926, in J. Med. Chem. **1985,** 28, (6), 717-727 oder in Arch. Pharm. **1990,** 323, (8), 501-505 beschrieben).

Die erhaltenen Verbindungen der Formel I können anschließend in ihre Salze, gegebenenfalls erhaltene Salze der Verbindungen der Formel I in freie Verbindungen übergeführt werden.

Gewünschtenfalls können erhaltene Verbindungen der Formel I auch in die entsprechenden N-Oxide übergeführt werden.

Die N-Oxidation erfolgt auf eine dem Fachmann ebenfalls vertraute Weise, z.B. mit Hilfe von m-Chlorperoxibenzoesäure in Dichlormethan bei Raumtemperatur. Welche Reaktionsbedingungen für die Durchführung des Verfahren im einzelnen erforderlich sind, ist dem Fachmann aufgrund seines Fachwissens geläufig.

Verbindungen der Formel II, worin Z eine geeignete Abgangsgruppe darstellt und R1, R2 und R3 die oben angegebenen Bedeutungen besitzen, lassen sich wie in den folgenden Beispielen beschrieben oder in einer dem Fachmann an sich vertrauten Weise aus den entsprechenden Verbindungen der Formel II, worin Z eine Hydroxygruppe bedeutet und R1, R2 und R3 die oben angegebenen Bedeutungen besitzen, herstellen.

Verbindungen der Formel II, worin Z eine Hydroxygruppe bedeutet und R1, R2 und R3 die oben angegebenen Bedeutungen besitzen, können wie in WO96/03399 beschrieben oder nach dem Fachmann vertrauten Methoden und Techniken erhalten werden.

Verbindungen der Formel III sind bekannt oder können in einer dem Fachmann an sich vertrauten Weise unter Anwendung üblicher Verfahren hergestellt werden.

Das zweite Verfahren (vgl. Schema 2) unterscheidet sich vom ersten Verfahren dadurch, daß im letzten Reaktionsschritt der Pyridinring und nicht wie im ersten Verfahren beschrieben, der Imidazolo- bzw. der Oxazoloring, aufgebaut wird.

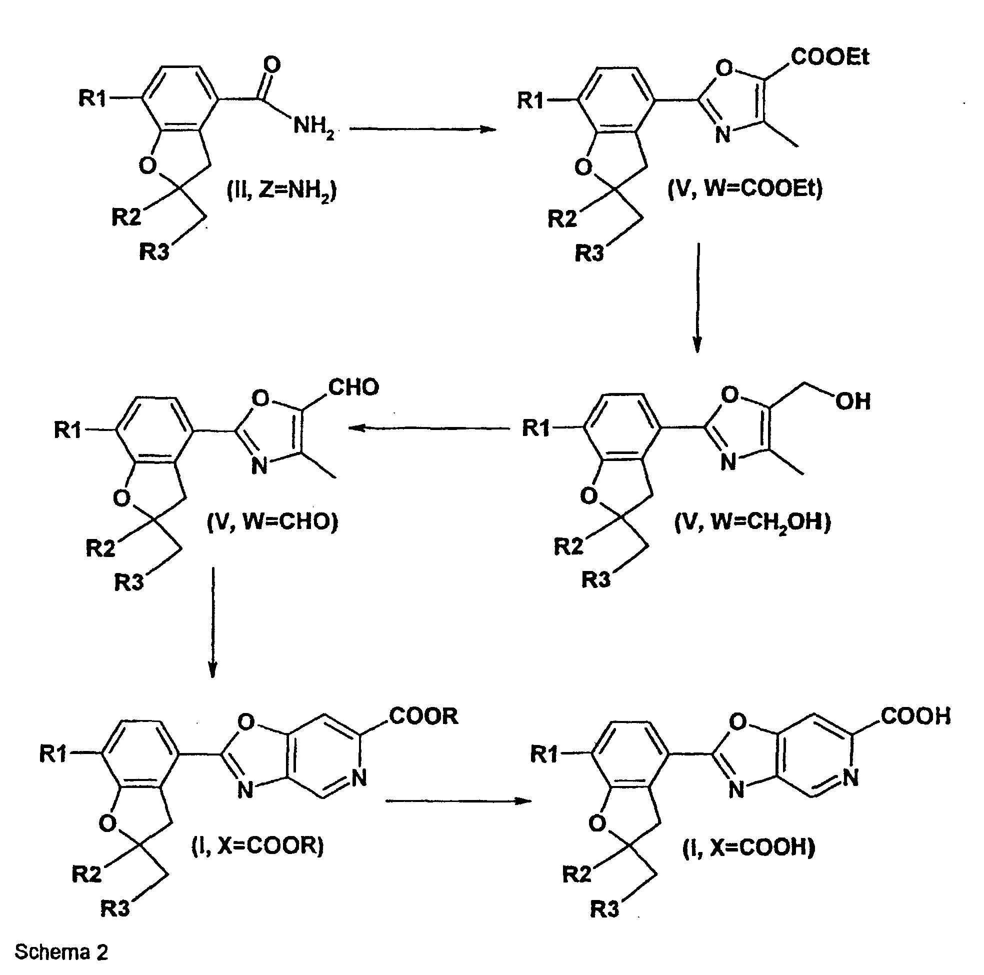

Das Verfahren eignet sich insbesondere zur Herstellung von Oxazolo[4,5-c]pyridin-Verbindungen, die in 6-Stellung eine Ester- oder Carboxylgruppe besitzen.

Das Verfahren ist dadurch gekennzeichnet, daß Verbindungen der Formel V, 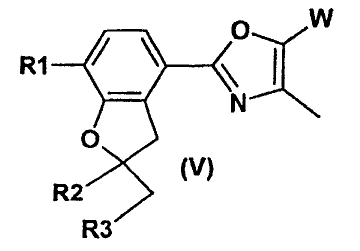 in denen R1, R2 und R3 die oben angegebenen Bedeutungen besitzen und W für CHO steht, mit Verbindungen der Formel N₃CH₂COOR, wobei R für 1-4C-Alkyl steht, umgesetzt werden.

Die Umsetzung erfolgt in zwei Schritten auf eine dem Fachmann an sich bekannte Weise, zum Beispiel so wie in den Beispielen beschrieben. Im ersten Schritt wird die Aldehydgruppe der Verbindungen der Formel V (W=CHO) mit der CH₂-Gruppe der α-Azidoester (N₃CH₂COOR) in Gegenwart einer geeigneten Base wie z.B. NaOR (R=1-4C-Alkyl) kondensiert. Im zweiten Schritt erfolgt durch thermische Behandlung in einem inerten Lösungsmittel, wie z.B. Xylol, der (Oxazolo-)Pyridin-Ringschluß.

Gewünschtenfalls können Verbindungen der Formel I, in denen R1, R2 und R3 die oben angegebenen Bedeutungen haben und X für COOR steht durch Anwendung dem Fachmann bekannter Methoden, beispielsweise durch Verseifung, in die entsprechenden Carbonsäuren der Formel I übergeführt werden.

Verbindungen der Formel N₃CH₂COOR sind bekannt oder können in einer dem Fachmann an sich vertrauten Weise unter Anwendung üblicher Verfahren hergestellt werden.

Verbindungen der Formel V, in denen R1, R2 und R3 die oben angegebenen Bedeutungen haben und W für CHO steht, können aus den entsprechenden Verbindungen der Formel V, in denen W für COOEt steht durch die Kombination einer Reduktion mit einer Oxidationsreaktion (W=COOEt → W=CH₂OH → W=CHO) hergestellt werden.

Verbindungen der Formel V, in denen R1, R2 und R3 die oben angegebenen Bedeutungen haben und W für COOEt steht, können aus den entsprechenden Verbindungen der Formel II, worin Z für NH₂ (Amino) steht, durch Umsetzung mit z.B. 2-Chloracetoessigsäureethylester hergestellt werden.

Verbindungen der Formel II, in denen R1, R2 und R3 die oben angegebenen Bedeutungen besitzen und Z für NH₂ (Amino) steht, können aus den entsprechenden Verbindungen der Formel II, in denen Z für eine geeignete Abgangsgruppe, beispielsweise ein Halogenatom steht, durch Umsetzung mit Ammoniak hergestellt werden.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niedermotekularen aliphatischen Alkohol (Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakoiogisch verträgliche Salze umwandeln.

Die nachfolgenden Beispiele dienen der näheren Erläuterung der Erfindung ohne sie einzuschränken. Ebenso können weitere Verbindungen der Formel I, deren Herstellung nicht explizit beschrieben ist, in analoger oder in einer dem Fachmann an sich vertrauten Weise unter Anwendung üblicher Verfahrenstechniken hergestellt werden.

in den Beispielen steht Schmp. für Schmelzpunkt, Sdp. für Siedepunkt, h für Stunde(n), RT für Raumtemperatur, SF für Summenformel und MG für Molgewicht. Die in den Beispielen genannten Verbindungen und ihre Salze sind bevorzugter Gegenstand der Erfindung.

### Beispiele

### Endprodukte

### 1. 2-(2,3-Dihydro-7-methoxy-benzofuran-2-spiro-1'-cyclopentan-4-yl)-oxazolo[4,5-b]pyridin

500 mg (1,5 mmol) N-(3-Hydroxy-pyridin-2-yl)-2,3-dihydro-7-methoxy-benzofuran-2-spiro-1'-cyclopentan-4-carbonsäureamid werden in 2 ml SOCl₂ 3 h bei 70°C gerührt. Überschüssiges SOCl₂ wird abdestilliert und der Rückstand über Kieselgel chromatographiert. Durch Kristallisation aus Diethylether erhält man die Titelverbindung. Schmp. 171 °C.

### 2. 2-(2,3-Dihydro-7-methoxy-benzofuran-2-spiro-1'-cyclopentan-4-yl)-oxazolo[5,4-b]pyridin

880 mg (2,6 mmol) N-(2-Hydroxy-pyridin-3-yl)-2,3-dihydro-7-methoxy-benzofuran-2-spiro-1'-cyclopentan-4-carbonsäureamid werden in 5 ml SOCl₂ 16 h bei 80°C gerührt. Überschüssiges SOCl₂ wird abdestilliert und der Rückstand mit einer 9:1-Mischung aus Toluol und Ethylacetat chromatographiert. Durch Kristallisation aus Diethylether erhält man die Titelverbindung. Schmp. 140°C.

### 3. 2-(2,3-Dihydro-7-methoxy-benzofuran-2-spiro-1'-cyclopentan-4-yl)-oxazolo[4,5-c]pyridin

370 mg (1,1 mmol) N-(4-Hydroxy-pyridin-3-yl)-2,3-dihydro-7-methoxy-benzofuran-2-spiro-1'-cyclopentan-4-carbonsäureamid werden in 3 ml SOCl₂ 6 h bei 80°C gerührt. Überschüssiges SOCl₂ wird abdestilliert und der Rückstand chromatographisch über Kieselgel mit einer 9:1 Mischung aus Toluol und Ethylacetat gereinigt. Schmp. 158°C.

### 4. 2-(2,3-Dihydro-7-methoxy-benzofuran-2-spiro-1'-cyclopentan-4-yl)-imidazo[4,5-c]pyridin

Das unter Ausgangsprodukt A4 erhaltene Amidgemisch wird in 10 ml POCl₃ gelöst und dann die Lösung 3 h bei 120°C gerührt. Nach Abkühlen wird auf Eiswasser gegossen, mit 10 N Natronlaugelösung auf pH 8 eingestellt und 3 x mit Ethylacetat extrahiert. Die vereinigten Extrakte werden über Natriumsulfat getrocknet, eingeengt und der Rückstand über eine Kieselgelsäule mit Ethylacetat /Methanol 9:1 als Laufmittel chromatografiert. Die chromatografisch reinen Fraktionen werden vereinigt, eingeengt und mit Petrolether kristallisiert. Schmp. 197-200°C.

### 5. 2-(2,3-Dihydro-7-methoxy-benzofuran-2-spiro-1'-cyclohexan-4-yl)-imidazo[4,5-c]pyridin

Analog Beispiel 4 wird das unter Ausgangsprodukt A5 erhaltene Amidgemisch mit POCl₃ cyclisiert. Nach Chromatografie mit Ethylacetat / Methanol 95:5 und Kristallisation mit Petrolether erhält man die Titelverbindung. Schmp. 183-185°C.

### 6. 2-(2,3-Dihydro-7-methoxy-benzofuran-2-spiro-1'-cyclohexan-4-yl)-imidazo[4,5-b]pyridin-dihydrochlorid-hemihydrat

Analog Beispiel 4 wird das unter Ausgangsprodukt A6 erhaltene Amid (1 g) mit POCl₃ cyclisiert. Nach Chromatografie mit Ethylacetat/Methanol 6:4 wird das Produkt mit überschüssiger etherischer Salzsäure in das Dihydrochlorid überführt, im Vakuum getrocknet und mit Diisopropylether kristallisiert. Schmp. 233-235°C.

### 7. 4-Chlor-2-(2,3-dihydro-7-methoxy-benzofuran-2-spiro-1'-cyclopentan-4yl)-imidazo[4,5-c]pyridin

Analog Beispiel 4 wird das unter Ausgangsprodukte A7 erhaltene Amid mit POCl₃ cyclisiert. Nach Chromatografie über eine Kieselgelsäule mit Ethylacetat als Laufmittel wird die Titelverbindung aus Diethylether kristallisiert. Schmp. 25B-260°C.

### 8. 2-(2,3-Dihydro-7-methoxy-benzofuran-2-spiro-1'-cyclopentan-4-yl)-oxazolo[4,5-c]pyridin-6-carbonsäure-methylester

2,5 g (8 mmol) 2-(2,3-Dihydro-7-methoxy-benzofuran-2-spiro-1'-cyclopentan-4-yl)-4-methyloxazol-5-carbaldehyd werden in 50 ml Methanol aufgeschlämmt und mit 5,7 ml 30 %iger Natriummethanolatlösung in Methanol versetzt. Unter Eiskühlung und Lichtausschluß werden 4,0 g (34 mmol) Azidoessigsäuremethylester zugetropft und 1 h bei 10-15°C gerührt. Anschließend wird das Reaktionsgemisch auf ges. Ammoniumchloridlösung gegeben und mit Dichlormethan extrahiert. Man trennt die Phasen, engt die organische Phase ein und trocknet über MgSO₄. Nach Abdampfen des Lösungsmittels wird in Methanol ausgerührt und die Zwischenverbindung im Hochvakuum getrocknet. Das getrocknete Azid wird in 100 ml Xylol 20 min auf 130°C erhitzt. Das Xylol wird im Vakuum verdampft und das Rohprodukt in Acetonitril umkristallisiert. Man erhält 470 mg der Titelverbindung vom Schmp. 194-195°C.

### 9. 2-(2,3-Dihydro-7-methoxy-benzofuran-2-spiro-1'-cyclopentan-4-yl)-oxazolo[4,5-c]pyridin-6-carbonsäure

400 mg (1 mmol) 2-(2,3-Dihydro-7-methoxy-benzofuran-2-spiro-1'-cyclopentan-4-yl)-oxazolo[4,5-c]pyridin-6-carbonsäure-methylester werden in 100 mg (4,0 mmol) Lithiumhydroxid In 20 ml einer 1:1 Mischung aus Methanol und Wasser bei RT über Nacht verseift. Das Methanol wird im Vakuum verdampft, die Reaktionslösung mit 20 ml Wasser versetzt und mit 4 ml 2N H₂SO₄ angesäuert. Der Kristallbrei wird mit 50 ml Wasser gewaschen und Im Hochvakuum getrocknet. Schmp. 200°C (Zers.).

### Ausgangsprodukte

### A1. N-(3-Hydroxy-pyridin-2-yl)-2,3-dihydro-7-methoxy-benzofuran-2-spiro-1'-cyclopentan-4-carbonsäureamid

1,5 g (6,1 mmol) 2,3-Dihydro-7-methoxy-benzofuran-2-spiro-1'-cyclopentan-4-carbonsäure werden mit 6,1 g (51,3 mmol) SOCl₂ 1,5 h am Rückfluß gekocht. Überschüssiges SOCl₂ wird abdestilliert und der Rückstand durch mehrmaliges koevaporieren mit Toluol vom restlichen SOCl₂ befreit. Das Säurechlorid wird in 50 ml Pyridin gelöst und mit 680 mg (6,2 mmol) 2-Amino-3-hydroxy-pyridin versetzt. Nach 5 h bei 80°C wird das Pyridin abdestilliert und der Rückstand mit CH₂Cl₂ aus Wasser extrahiert. Die getrocknete organische Phase wird eingeengt und das Rohprodukt aus Diethylether kristallisiert. Man erhält 1,08 g der Titelverbindung. Schmp. 162°C.

### A2. N-(2-Hydroxy-pyridin-3-yl)-2,3-dihydro-7-methoxy-benzofuran-2-spiro-1'-cyclopentan-4-carbonsäureamid

Die Titelverbindung wird erhalten, wenn analog Beispiel A1 3-Amino-2-hydroxy-pyridin als Ausgangsverbindung eingesetzt wird. Schmp. 235-240°C.

### A3. N-(4-Hydroxy-pyridin-3-yl)-2,3-dihydro-7-methoxy-benzofuran-2-spiro-1'-cyclopentan-4-carbonsäureamid

Die Titelverbindung wird erhalten, wenn analog Beispiel A1 3-Amino-4-hydroxypyridin als Ausgangsverbindung eingesetzt wird.

### A4. N-(4-Aminopyridin-3-yl)- und N-(3-Aminopyridin-4-yl)-2,3-dihydro-7-methoxy-benzofuran-2-spiro-1'-cyclopentan-4-carbonsäureamid

1,8 g 2,3-Dihydro-7-methoxy-benzofuran-2-spiro-1'-cyclopentan-4-carbonsäure werden in 50 ml Toluol gelöst, mit 5 ml SOCl₂ versetzt und das Gemisch 1 h unter Rückfluß zum Sieden erhitzt. Nach Abkühlen wird das Toluol Im Vakuum am Rotationsverdampfer abdestilliert, zur vollständigen Entfernung des überschüssigen SOCl₂ noch 2 x mit Toluol nachdestilliert und der Rückstand im 20 ml absolutem Dioxan gelöst. Die Lösung wird unter Rühren bei 40°C zu einer Lösung von 1,1 g 3,4-Diaminopyridin und 1,3 ml Triethylamin in 100 ml absolutem Dioxan zugetropft. Es wird noch 2 h bei 40°C gerührt, dann wird auf Wasser gegossen und 3 x mit Ethylacetat extrahiert. Die vereinigten Extrakte werden über Natriumsulfat getrocknet und eingeengt. Das so erhaltene Gemisch der Titelverbindungen wird ohne weitere Reinigung weiter umgesetzt.

### A5. N-(4-Aminopyridin-3-yl)- und N-(3-Amino-pyridin-4-yl)-2,3-dihydro-7-methoxy-benzofuran-2-spiro-1'-cyclohexan-4-carbonsäureamid

Analog Ausgangsprodukt A4 wird aus 2 g 2,3-Dihydro-7-methoxy-benzofuran-2-spiro-1'-cyclohexan-4-carbonsäure das Säurechlorid hergestellt und mit 0,83 g 3,4-Diaminopyridin und 1,0 ml Triethylamin zum Säureamidgemisch umgesetzt, das ohne weitere Reinigung weiter umgesetzt wird.

### A6. N-(2-Aminopyridin-3-yl)-2,3-dihyrdro-7-methoxy-benzofuran-2-spiro-1'-cyclohexan-4-carbonsäureamid

Analog Ausgangsprodukt A4 wird aus 2 g 2,3-Dihydro-7-methoxy-benzofuran-2-spiro-1'-cyclohexan-4-carbonsäure das Säurechlorid hergestellt und mit 0,83 g 2,3-Diaminopyridin und 1,0 ml Triethylamin zum Säureamid umgesetzt. Das Rohprodukt wird über eine Kieselgelsäule mit Ethylacetat als Laufmittel chromatografiert. Die chromatografisch reinen Fraktionen werden vereinigt und eingeengt. Man erhält 1 g der Titelverbindung als gelbes, zähes Öl.

### A7. N-(4-Amino-2-chlorpyridin-3-yl)-2,3-dihydro-7-methoxy-benzofuran-2-spiro-1'-cyclopentan-4-carbonsäureamid

Analog Ausgangsprodukt A4 wird aus 0,7 g 2,3-Dihydro-7-methoxy-benzofuran-2-spiro-1'-cyclopentan-4-carbonsäure das Säurechlorid hergestellt und mit 0,4 g 3,4-Diamino-2-chlorpyridin und 0,4 ml Triethylamin umgesetzt. Das Rohprodukt wird ohne weitere Reinigung weiter umgesetzt.

### A8. 2-(2,3-Dihydro-7-methoxy-benzofuran-2-spiro-1'-cyclopentan-4-yl)-4-methyl-oxazol-5-carbaldehyd

7,38 g (23 mmol) 2-(2,3-Dihydro-7-methoxy-benzofuran-2-spiro-1'-cyclopentan-4-yl)-4-methyl-oxazol-5-yl-methanol werden in 200 ml Dichlormethan bei RT über 24 h portionsweise mit 15 g Mangandioxid versetzt. Das Reaktionsgemisch wird über Kieselgur filtriert, das Filtrat eingeengt und der Rückstand aus 2-Propanol kristallisiert. Man erhält 5,1 g der Titelverbindung.

### A9. 2-(2,3-Dihydro-7-methoxy-benzofuran-2-spiro-1'-cyclopentan-4-yl)-4-methyl-oxazol-5-ylmethanol

8,9 g (28 mmol) 2-(2,3-Dihydro-7-methoxy-benzofuran-2-spiro-1'-cyclopentan-4-yl)-4-methyl-oxazol-5-carbonsäure-ethylester in 100 ml THF werden bei 60°C in eine Suspension von 1,9 g (50 mmol) LiAlH₄ in 40 ml THF getropft. Nach 3 h Kochen am Rückfluß wird mit 10 ml Wasser langsam hydrolysiert, mit 1,9 ml 4N NaOH versetzt, filtriert und die organische Phase des Filtrats abgetrennt, über MgSO₄ getrocknet und eingeengt. Der Rückstand wird in Petrolether ausgerührt. Man erhält 6,6 g der Titelverbindung.

### A10. 2-(2,3-Dihydro-7-methoxy-benzofuran-2-spiro-1'-cyclopentan-4-yl)-4-methyl-oxazol-5-carbonsäure-ethylester

15 g (61 mmol) 2-(2,3-Dihydro-7-methoxy-benzofuran-2-spiro-1'-cyclopentan-4-yl)-4-benzamid wird in 25 ml (121 mmol) 95 %igem 2-Chlor-acetoessigsäure-ethylester aufgeschlämmt und unter Rühren portionsweise 3,6 g (64 mmol) Calciumoxid zugegeben. Man kocht 4 Tage bei 120°C. Anschließend wird mit 15 ml halbkonz. HCl, 300 ml Wasser und 100 ml Ethylacetat versetzt. Man trennt die Phasen und extrahiert zweimal mit je 100 ml Ethylacetat. Die vereinigten organischen Phasen werden getrocknet (MgSO₄) und vom Lösungsmittel befreit. Man chromatographiert über Kieselgel (Toluol/Ethylacetat 40:1) und isoliert 6,9 g der Titelverbindung.

### A11. 2-(2,3-Dihydro-7-methoxy-benzofuran-2-spiro-1'-cyclopentan-4-yl)-4-benzamid

3,5 g (14,0 mmol) 2,3-Dihydro-7-methoxy-benzofuran-2-spiro-1'-cyclopentan-4-yl-carbonsäure werden mit 10 ml (ca. 140 mmol) SOCl₂ behandelt. Das überschüssige SOCl₂ wird im Vakuum abdestilliert und der Rückstand in 20 ml Aceton aufgenommen. Man versetzt unter Eiskühlung mit 10 ml konz. NH₃ und läßt 1 h nachrühren. Das Aceton wird abdestilliert und der Rückstand zwischen Ethylacetat und 0,5 N NaOH verteilt. Die getrocknete organische Phase wird aus 5 ml 50 %igem Methanol kristallisiert und man erhält 115 mg der Titelverbindung vom Schmp. 149-151°C.

### Gewerbliche Anwendbarkeit

Die erfindungsgemäßen Verbindungen besitzen wertvolle pharmakologische Eigenschaften, die sie gewerblich verwertbar machen. Als selektive Zyklisch-Nukleotid Phosphodiesterase (PDE) Inhibitoren (und zwar des Typs 4) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigernden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des Darms, der Augen, des zentralen Nervensystems und der Gelenke, die vermittelt werden durch Mediatoren, wie Histamin, PAF (Plättchen-aktivierender Faktor), Arachidonsäure-Abkömmlinge wie Leukotriene und Prostaglandine, Zytokine, Interleukine, Chemokine, alpha-, beta- und gamma-Interferon, Tumomekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Harnleiter im Zusammenhang mit Nierensteinen, oder auch Erkrankungen des ZNS, wie beispielsweise Depressionen oder arteriosklerotische Demenz.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 1 mg pro Sprühstoß. Die übliche Dosis bei systemischer Therapie (p.o. oder i.v.) liegt zwischen 0,1 und 200 mg pro Applikation.

### Biologische Untersuchungen

Bei der Untersuchung der PDE 4-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminol-verstärkte Chemolumineszenz gemessen werden kann. [Mc Phail LC, Strum SL, Leone PA und Sozzani S, The neutrophil respiratory burst mechanism. In "Immunology Series" **1992**, 57, 47-76; ed. Coffey RG (Marcel Decker, Inc., New York-Basel-Hong Kong)].

Substanzen, welche die Chemolumineszenz sowie die Zytokinsekretion und die Sekretion entzündungssteigemder Mediatoren an Entzündungszellen, Insbesonders neutrophilen und eosinophilen Granulozyten, T-Lymphozyten, Monozyten und Makrophagen hemmen, sind solche, welche die PDE 4 hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE 4-Hemmung durch die erfindungsgemäßen Substanzen ist damit ein zentraler Indikator für die Unterdrückung von entzündlichen Prozessen. (**Giembycz MA**, Could isoenzyme-selective phosphodiesterase inhibitors render bronchodilatory therapy redundant in the treatment of bronchial asthma?. Biochem Pharmacol **1992,** 43, 2041-2051; **Torphy TJ** et al., Phosphodiesterase inhibitors: new opportunities for treatment of asthma. Thorax **1991,** 46, 512-523; **Schudt C** et al., Zardaverine: a cyclic AMP PDE 3/4 Inhibitor. In "New Drugs for Asthma Therapy", 379-402, Birkhäuser Verlag Basel 1991; **Schudt C** et al., Influence of selective phosphodiesterase inhibitors on human neutrophil functions and levels of cAMP and Ca; Naunyn-Schmiedebergs Arch Pharmacol **1991,** 344, 682-690; **Nielson CP** et al., Effects of selective phosphodiesterase inhibitors on polymorphonuclear leukocyte respiratory burst. J Allergy Clin Immunol **1990,** 86, 801-808; **Schade** et al., The specific type 3 and 4 phosphodiesterase inhibitor zardaverine suppress formation of tumor necrosis factor by macrophages. European Joumal of Pharmacology **1993,** 230, 9-14).

### Hemmung der PDE4-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. **1980,** 311, 193-198). Hierbei erfolgt im ersten Schritt die PDE-Reaktion. In einem zweiten Schritt wird das entstandene 5'-Nukteotid durch eine 5'-Nukleotidase des Schlangengiftes von Crotalus Atrox zum ungeladenen Nukleosid gespalten. Im dritten Schritt wird das Nukleosid auf lonenaustauschsäulen vom verbliebenen geladenen Substrat getrennt. Die Säulen werden mit 2 ml 30 mM Ammoniumformiat (pH 6,0) direkt in Minivials eluiert, in die noch 2 ml Szintillatorfiüssigkeit zur Zählung gegeben wird.

Die für die erfindungsgemäßen Verbindungen ermittelten Hemmwerte [Hemmkonzentration als - log IC₅₀ (mol/l)] ergeben sich aus der folgenden Tabelle A, in der die Nummern der Verbindungen den Nummern der Beispiele entsprechen.

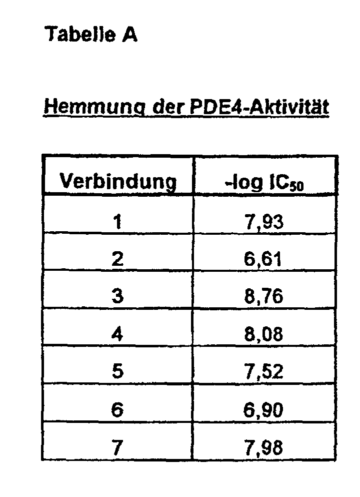

## Patentansprüche

1. Verbindungen der Formel I, 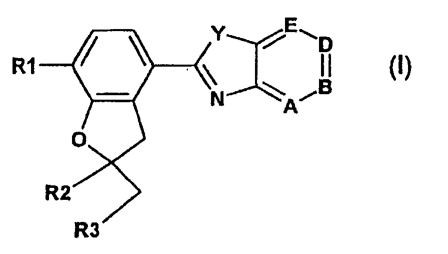 worin
aus der Gruppe der Symbole A, B, D und E unabhängig voneinander ein Symbol für N (Stickstoff) steht, ein Symbol für C-X und die verbleibenden zwei Symbole für C-H stehen, wobei
X Wasserstoff. Hydroxy, Halogen, Cyano, Nitro, Carboxyl, 1-4C-Alkyl, 1-4C-Alkylcarbonyl, 1-4C-Alkylcarbonyloxy, 1-4C-Alkoxy, 1-4C-Alkoxycarbonyl, Amino, Mono- oder Di-1-4C-alkylamino, 1-4C-Alkylcarbonylamino, Hydroxy-1-4C-alkyl, Hydroxysulfonyl, 1-4C-Alkylsulfonyl, 1-4C-Alkoxysulfonyl oder Sulfamoyl bedeutet,
Y O (Sauerstoff) oder NH bedeutet,
R1 1-4C-Alkoxy, 3-7C-Cycloalkoxy, 3-7C-Cycloalkylmethoxy, Benzyloxy oder ganz oder überwiegend durch Fluor substituiertes 1-4C-Alkoxy bedeutet,
R2 Wasserstoff oder 1-4C-Alkyl und
R3 Wasserstoff oder 1-4C-Alkyl bedeuten,
oder worin
R2 und R3 gemeinsam und unter Einschluß der beiden Kohlenstoffatome, an die sie gebunden sind, einen spiro-verknüpften 5-, 6- oder 7-gliedrigen, gewünschtenfalls durch ein Sauerstoffatom unterbrochenen Kohlenwasserstoffring darstellen,
sowie die Salze und die N-Oxide dieser Verbindungen.

2. Verbindungen der Formel I nach Anspruch 1, in denen
aus der Gruppe der Symbole A, B, D und E unabhängig voneinander ein Symbol für N (Stickstoff) steht, ein Symbol für C-X und die verbleibenden zwei Symbole für C-H stehen, wobei
X Wasserstoff, Hydroxy, Halogen, Cyano, Nitro, Carboxyl, 1-4C-Alkyl, 1-4C-Alkylcarbonyloxy, 1-4C-Alkoxy, 1-4C-Alkoxycarbonyl, Amino, Mono- oder Di-1-4C-alkylamino, 1-4C-Alkylcarbonylamino, Hydroxy-1-4C-alkyl, Hydroxysulfonyl oder Sulfamoyl bedeutet,
Y O oder NH bedeutet,
R1 1-4C-Alkoxy, 3-7C-Cycloalkoxy, 3-7C-Cycloalkylmethoxy oder ganz oder überwiegend durch Fluor substituiertes 1-4C-Alkoxy bedeutet,
R2 1-4C-Alkyl und
R3 Wasserstoff oder 1-4C-Alkyl bedeuten,
oder worin
R2 und R3 gemeinsam und unter Einschluß der beiden Kohlenstoffatome, an die sie gebunden sind, einen spiro-verknüpften Cyclopentan-, Cyclohexan-, Tetrahydrofuran- oder Tetrahydropyranring darstellen,
sowie die Salze dieser Verbindungen.

3. Verbindungen der Formel I nach Anspruch 1, in denen
aus der Gruppe der Symbole A, B, D und E unabhängig voneinander ein Symbol für N (Stickstoff) steht, ein Symbol für C-X und die verbleibenden zwei Symbole für C-H stehen, wobei
X Wasserstoff, Halogen, Carboxyl oder 1-4C-Alkoxycarbonyl bedeutet,
Y O oder NH bedeutet,
R1 Methoxy, Ethoxy, Cyclopropylmethoxy oder ganz oder überwiegend durch Fluor substituiertes 1-2C-Alkoxy bedeutet,
R2 1-4C-Alkyl und
R3 Wasserstoff oder 1-4C-Alkyl bedeuten,
oder worin
R2 und R3 gemeinsam und unter Einschluß der beiden Kohlenstoffatome, an die sie gebunden sind, einen spiro-verknüpften Cyclopentan- oder Cyclohexanring darstellen,
sowie die Salze dieser Verbindungen.

4. Verbindungen der Formel I nach Anspruch 1, in denen
aus der Gruppe der Symbole A, B, D und E unabhängig voneinander ein Symbol für N (Stickstoff) steht, ein Symbol für C-X und die verbleibenden zwei Symbole für C-H stehen, wobei
X Wasserstoff oder Halogen bedeutet,
Y O oder NH bedeutet,
R1 Methoxy bedeutet und
R2 und R3 gemeinsam und unter Einschluß der beiden Kohlenstoffatome, an die sie gebunden sind, einen spiro-verknüpften Cyclopentan- oder Cyclohexanring darstellen,
sowie die Salze dieser Verbindungen.

5. Arzneimittel enthaltend eine oder mehrere Verbindungen nach Anspruch 1 zusammen mit üblichen pharmazeutischen Hilfs- und/oder Trägerstoffen.

6. Verbindungen nach Anspruch 1 zur Anwendung bei der Behandlung von Krankheiten.

7. Verwendung von Verbindungen nach Anspruch 1 zur Herstellung von Arzneimitteln für die Behandlung von Atemwegserkrankungen.

8. Verwendung von Verbindungen nach Anspruch 1 zur Herstellung von Arzneimitteln für die Behandlung von Dermatosen.

## Claims

1. Compounds of formula I 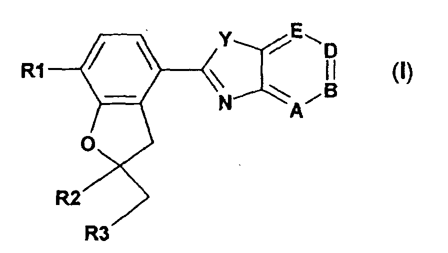 in which
of the group of symbols A, B, D and E, independently of one another, one symbol is N (nitrogen), one symbol is C-X and the remaining two symbols are C-H, where
X is hydrogen, hydroxyl, halogen, cyano, nitro, carboxyl, 1-4C-alkyl, 1-4C-alkylcarbonyl, 1-4C-alkylcarbonyloxy, 1-4C-alkoxy, 1-4C-alkoxycarbonyl, amino, mono- or di-1-4C-alkylamino, 1-4C-alkylcarbonylamino, hydroxy-1-4C-alkyl, hydroxysulfonyl, 1-4C-alkylsulfonyl, 1-4C-alkoxysulfonyl or sulfamoyl,
Y is O (oxygen) or NH,
R1 is 1-4C-alkoxy, 3-7C-cydoalkoxy, 3-7C-cycloalkylmethoxy, benzyloxy or 1-4C-alkoxy which is completely or predominantly substituted by fluorine,
R2 is hydrogen or 1-4C-alkyl and
R3 is hydrogen or 1-4C-alkyl,
or in which
R2 and R3, together and including the two carbon atoms to which they are bonded, are a spiro-linked 5-, 6- or 7-membered hydrocarbon ring, if desired interrupted by an oxygen atom,
and the salts and the N-oxides of these compounds.

2. Compounds of formula I as claimed in claim 1, in which
from the group of symbols A, B, D and E, independently of one another, one symbol is N (nitrogen), one symbol is C-X and the remaining two symbols are C-H, where
X is hydrogen, hydroxyl, halogen, cyano, nitro, carboxyl, 1-4C-alkyl, 1-4C-alkylcarbonyloxy, 1-4C-alkoxy, 1-4C-alkoxycarbonyl, amino, mono- or di-1-4C-alkylamino, 1-4C-alkylcarbonylamino, hydroxy-1-4C-alkyl, hydroxysulfonyl or sulfamoyl,
Y is O or NH,
R1 is 1-4C-alkooy, 3-7C-cycloalkoxy, 3-7C-cycloalkylmethoxy or 1-4C-alkoxy which is completely or predominantly substituted by fluorine,
R2 is 1-4C-alkyl and
R3 is hydrogen or 1-4C-alkyl,
or in which
R2 and R3, together and including the two carbon atoms to which they are bonded, are a spiro-linked
cyclopentane, cyclohexane, tetrahydrofuran or tetrahydropyran ring,
and the salts of these compounds.

3. Compounds of formula I as claimed in claim 1, in which
from the group of symbols A, B, D and E, independently of one another, one symbol is N (nitrogen), one symbol is C-X and the remaining two symbols are C-H, where
X is hydrogen, halogen, carboxyl or 1-4C-alkoxycarbonyl,
Y is O or NH,
R1 is methoxy, ethoxy, cyclopropylmethoxy or 1-2C-alkoxy which is completely or predominantly substituted by fluorine,
R2 is 1-4C-alkyl and
R3 is hydrogen or 1-4C-alkyl,
or in which
R2 and R3, together and including the two carbon atoms to which they are bonded, are a spiro-linked cyclopentane or cyclohexane ring,
and the salts of these compounds.

4. Compounds of formula I as claimed in claim 1, in which
from the group of symbols A, B, D and E, independently of one another, one symbol is N (nitrogen), one symbol is C-X and the remaining two symbols are C-H, where
X is hydrogen or halogen,
Y is O or NH,
R1 is methoxy and
R2 and R3, together and including the two carbon atoms to which they are bonded, are a spiro-linked cyclopentane or cyclohexane ring,
and the salts of these compounds.

5. A pharmaceutical composition comprising one or more compounds as claimed in claim 1 together with customary pharmaceutical auxiliaries and/or excipients.

6. A compound as claimed in claim 1 for use in the treatment of diseases.

7. The use of compounds as claimed in claim 1 for the production of pharmaceutical compositions for the treatment of airway disorders.

8. The use of compounds as claimed in claim 1 for the production of pharmaceutical compositions for the treatment of dermatoses.

## Revendications

1. Composés de formule I 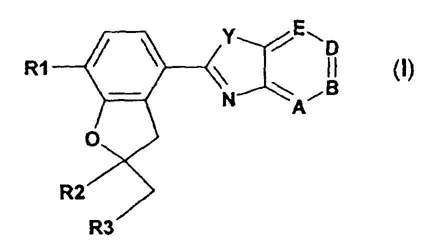 où
dans le groupe des symboles A, B, D et E, indépendamment les uns des autres un symbole représente N (azote), un symbole représente C-X et les deux symboles restants représentent C-H, où
X représente l'hydrogène, hydroxyle, halogène, cyano, nitro, carboxyle, alkyle en C1-4, alkyle en C1-4-carbonyle, alkyle en C1-4-carbonyloxy, alcoxy en C1-4, alcoxy en C1-4-carbonyle, amino, mono- ou di-alkyle en C1-4-amino, alkyle en C1-4-carbonylamino, hydroxy-alkyle en C1-4, hydroxysulfonyle, alkyle en C1-4-sulfonyle, alcoxy en C1-4-sulfonyle ou sulfamoyle,
Y représente O (oxygène) ou NH,
R1 représente alcoxy en C1-4, cycloalcoxy en C3-7, cycloalkyle en C3-7-méthoxy, benzyloxy ou alcoxy en C1-4 substitué totalement ou majoritairement par le fluor,
R2 représente l'hydrogène ou alkyle en C1-4 et
R3 représente l'hydrogène ou alkyle en C1-4,
ou bien où
R2 et R3 représentent ensemble et en incluant les deux atomes de carbone auxquels ils sont liés un cycle hydrocarboné à 5, 6 ou 7 chaînons, à liaison spiro, interrompu si on le souhaite par un atome d'oxygène,
ainsi que les sels et les N-oxydes de ces composés.

2. Composés de formule I selon la revendication 1 dans lesquels
dans le groupe des symboles A, B, D et E, indépendamment les uns des autres un symbole représente N (azote), un symbole représente C-X et les deux symboles restants représentent C-H, où
X représente l'hydrogène, hydroxyle, halogène, cyano, nitro, carboxyle, alkyle en C1-4, alkyle en C1-4-carbonyloxy, alcoxy en C1-4, alcoxy en C1-4-carbonyle, amino, mono- ou di-alkyle en C1-4-amino, alkyle en C1-4-carbonylamino, hydroxy-alkyle en C1-4, hydroxysulfonyle ou sulfamoyle,
Y représente 0 ou NH,
R1 représente alcoxy en C1-4, cycloalcoxy en C3-7, cycloalkyle en C3-7-méthoxy ou alcoxy en C1-4 substitué totalement ou majoritairement par le fluor,
R2 représente alkyle en C1-4 et
R3 représente l'hydrogène ou alkyle en C1-4,
ou bien où
R2 et R3 représentent ensemble et en incluant les deux atomes de carbone auxquels ils sont liés un cycle cyclopentane, cyclohexane, tétrahydrofurane ou tétrahydropyrane à liaison spiro,
ainsi que les sels de ces composés.

3. Composés de formule I selon la revendication 1 dans lesquels
dans le groupe des symboles A, B, D et E, indépendamment les uns des autres un symbole représente N (azote), un symbole représente C-X et les deux symboles restants représentent C-H, où
X représente l'hydrogène, halogène, carboxyle ou alcoxy en C1-4-carbonyle,
Y représente O ou NH,
R1 représente méthoxy, éthoxy, cyclopropylméthoxy ou alcoxy en C1-2 substitué totalement ou majoritairement par le fluor,
R2 représente alkyle en C1-4 et
R3 représente l'hydrogène ou alkyle en C1-4,
ou bien où
R2 et R3 représentent ensemble et en incluant les deux atomes de carbone auxquels ils sont liés un cycle cyclopentane ou cyclohexane à liaison spiro,
ainsi que les sels de ces composés.

4. Composés de formule I selon la revendication 1 dans lesquels
dans le groupe des symboles A, B, D et E, indépendamment les uns des autres un symbole représente N (azote), un symbole représente C-X et les deux symboles restants représentent C-H, où
X représente l'hydrogène ou halogène,
Y représente O ou NH,
R1 représente méthoxy et
R2 et R3 représentent ensemble et en incluant les deux atomes de carbone auxquels ils sont liés un cycle cyclopentane ou cyclohexane à liaison spiro,
ainsi que les sels de ces composés.

5. Médicament contenant un ou plusieurs composés selon la revendication 1 en même temps que des adjuvants et/ou supports pharmaceutiques courants.

6. Composés selon la revendication 1 destinés à être utilisés dans le traitement de maladies.

7. Utilisation de composés selon la revendication 1 pour la production de médicaments pour le traitement de maladies des voies respiratoires.

8. Utilisation de composés selon la revendication 1 pour la production de médicaments pour le traitement de dermatoses.
